(19) **Europäisches Patentamt**
**European Patent Office**
**Office européen des brevets**

(11) **EP 4 621 439 A1**

(12) **EUROPEAN PATENT APPLICATION**

(43) Date of publication:
**24.09.2025 Bulletin 2025/39**

(21) Application number: **24193031.2**

(22) Date of filing: **06.08.2024**

(51) International Patent Classification (IPC):
**G01S 13/58** (2006.01)    **G01S 13/88** (2006.01)
**G08B 21/04** (2006.01)

(52) Cooperative Patent Classification (CPC):
**G01S 13/88; G01S 13/582; G01S 13/584;
G08B 21/043**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.03.2024 KR 20240037838**

(71) Applicant: **Bitsensing Inc.
Seongnam-si, Gyeonggi-do 13105 (KR)**

(72) Inventors:
• **CHOE, Sun Taag
16413 Suwon-si, Gyeonggi-do (KR)**
• **CHOI, Jin Gul
13597 Seongnamsi, Gyeonggi-do (KR)**
• **NAM, Seong Ung
13515 Seongnam-si, Gyeonggi-do (KR)**

(74) Representative: **BCKIP Part mbB
MK1
Landsbergerstraße 98, 3.Stock
80339 München (DE)**

(54) **DEVICE AND METHOD OF DETECTING FALL**

(57)    A fall detection device (100) comprising a transceiver (210) configured to transmit a radar signal toward a subject and receive a radar signal reflected from the subject; an observation determination unit (230) configured to determine whether or not to enter an observation state of the subject by analyzing the received radar signal; and a fall determination unit (240) configured to derive a range value between the transceiver and the subject in the observation state and determine whether the subject has fallen based on the range value.

# FIG. 2

Description

TECHNICAL FIELD

The present disclosure relates to a device and method of detecting a fall of a subject using a radar signal.

BACKGROUND

People of all ages may experience falls, and as society evolves into an aging society, the occurrence of falls among the elderly is increasing. In particular, elderly people who live alone may be accompanied by serious injury or death due to falls. Therefore, it is important to rapidly detect a fall and appropriately deal with the fall.

The conventional sensor technology used for detecting a fall includes an acceleration sensor, a tilt sensor, a geomagnetic sensor, a camera, and the like. Among them, the acceleration sensor and the tilt sensor need to be attached to the body of a user or held by the user in order to detect a rapid movement or a posture change at the time of a fall, which may cause the user inconvenience and impose a burden on the user. Also, the geomagnetic sensor operates in a similar manner, and all of these sensors need to be attached to the body of the user or held by the user at all times. A camera-based detection method may cause a critical problem in protecting personal privacy. The camera can detect a fall relatively accurately, but is restricted in use particularly in a private space due to possible invasion of privacy.

Such conventional sensors provide predetermined functions for detecting a fall, but have room for improvement in terms of convenience in use, protection of privacy of the user, accuracy in detection, and reliability. Wearing or holding a sensor at all times imposes a heavy burden, particularly, on the elderly or users who have difficulty in moving, which can be a factor hindering persistent use of a fall detection system. Accordingly, there is a need to develop a new method of detecting a fall which can overcome the limitations of the conventional methods and provides high accuracy in detection with higher user-friendliness and respect for privacy.

Radar technology can be used to detect a fall by detecting the range and velocity of a target. However, a radar sensor can detect all the moving objects, and, thus, a technology for differentiating movements relevant to a fall is indispensable in environments, such as bathroom, where movements of water can be confused with a fall. Conventional radar-based fall detection methods are limited in differentiating the movements in such environments. Accordingly, there is a need for a more sophisticated method and algorithm for detecting a fall.

SUMMARY

In view of the foregoing, the present disclosure is conceived to provide a fall detection device capable of accurately detecting a signal relevant to a fall from a radar signal.

The present disclosure is conceived to provide a device capable of protecting privacy of a target and detecting the presence or absence of a fall by detecting a fall using a radar signal.

The present disclosure is conceived to provide a device capable of detecting the presence or absence of a fall by detecting a fall using a radar signal even when a user does not wear or hold the detection device.

The problems to be solved by the present disclosure are not limited to the above-described problems. There may be other problems to be solved by the present disclosure.

An aspect of the present disclosure provides a device of detecting a fall using a radar signal, including: a transceiver configured to transmit a radar signal toward a subject and receive a radar signal reflected from the subject; an observation determination unit configured to determine whether or not to enter an observation state of the subject by analyzing the received radar signal; and a fall determination unit configured to derive a range value between the transceiver and the subject in the observation state and determine whether the subject has fallen based on the range value.

This summary is provided by way of illustration only and should not be construed as limiting in any manner. Besides the above-described exemplary embodiments, there may be additional exemplary embodiments that become apparent by reference to the drawings and the detailed description that follows.

According to an embodiment of the present disclosure, it is possible to differentiate and detect a signal relevant to a fall from a radar signal. Thus, it is possible to improve the accuracy in detecting a fall.

According to an embodiment of the present disclosure, it is possible to detect a fall using a radar signal. Thus, it is possible to detect the presence or absence of a fall while protecting privacy without directly imaging a target or recording sounds.

According to an embodiment of the present disclosure, it is possible to detect a fall using a radar signal. Thus, it is possible to detect the presence or absence of a fall even when a user does not wear or hold a detection device. Therefore, it is possible to improve the convenience in detecting a fall and reduce a burden on the user.

BRIEF DESCRIPTION OF THE DRAWINGS

In the detailed description that follows, embodiments are described as illustrations only since various changes and modifications will become apparent to a person with ordinary skill in the art from the following detailed description. The use of the same reference numbers in different figures indicates similar or identical items.

    FIG. 1 is a configuration diagram of a fall detection system according to an embodiment of the present disclosure.

**FIG. 2** is a configuration diagram of a fall detection device according to an embodiment of the present disclosure.

**FIG. 3** is a diagram explaining a procedure of generating range-Doppler map information according to an embodiment of the present disclosure.

**FIG. 4A** and **FIG. 4B** are diagrams explaining a procedure of deriving a peak signal according to an embodiment of the present disclosure.

**FIG. 5A** and **FIG. 5B** are diagrams explaining a procedure of deriving a movement peak signal according to an embodiment of the present disclosure.

**FIG. 6** is a diagram explaining a procedure of analyzing a pattern of movement peak signals according to an embodiment of the present disclosure.

**FIG. 7A** and **FIG. 7B** are diagrams explaining a procedure of deriving a trend line according to an embodiment of the present disclosure.

**FIG. 8** is a diagram explaining a procedure of deriving a range value according to an embodiment of the present disclosure.

**FIG. 9** and **FIG. 10** are diagrams explaining a procedure of determining the presence or absence of a fall according to an embodiment of the present disclosure.

**FIG. 11** to **FIG. 13** are flowcharts showing a fall detection method according to an embodiment of the present disclosure.

**FIG. 14** and **FIG. 15** are diagrams explaining parameters used in the procedure of determining the presence or absence of a fall according to an embodiment of the present disclosure.

**FIG. 16** is a flowchart showing a fall detection method according to an embodiment of the present disclosure.

## DETAILED DESCRIPTION

Hereafter, example embodiments will be described in detail with reference to the accompanying drawings so that the present disclosure may be readily implemented by those skilled in the art. However, it is to be noted that the present disclosure is not limited to the example embodiments but can be embodied in various other ways. In the drawings, parts irrelevant to the description are omitted for the simplicity of explanation, and like reference numerals denote like parts through the whole document.

Throughout this document, the term "connected to" may be used to designate a connection or coupling of one element to another element and includes both an element being "directly connected" another element and an element being "electronically connected" to another element via another element. Further, it is to be understood that the terms "comprises," "includes," "comprising," and/or "including" means that one or more other components, steps, operations, and/or elements are not excluded from the described and recited systems, devices, apparatuses, and methods unless context dictates otherwise; and is not intended to preclude the possibility that one or more other components, steps, operations, parts, or combinations thereof may exist or may be added.

Throughout this document, the term "unit" may refer to a unit implemented by hardware, software, and/or a combination thereof. As examples only, one unit may be implemented by two or more pieces of hardware or two or more units may be implemented by one piece of hardware.

Throughout this document, a part of an operation or function described as being carried out by a terminal or device may be implemented or executed by a device connected to the terminal or device. Likewise, a part of an operation or function described as being implemented or executed by a device may be so implemented or executed by a terminal or device connected to the device.

The functionality of the elements disclosed herein may be implemented using circuitry or processing circuitry which includes general purpose processors, special purpose processors, integrated circuits, ASICs ("Application Specific Integrated Circuits"), conventional circuitry and/or combinations thereof which are configured or programmed to perform the disclosed functionality.

Processors are considered processing circuitry or circuitry as they include transistors and other circuitry therein. In the disclosure, the circuitry, units, or means are hardware that carry out or are programmed to perform the recited functionality.

The hardware may be any hardware disclosed herein or otherwise known which is programmed or configured to carry out the recited functionality. When the hardware is a processor which may be considered a type of circuitry, the circuitry, means, or units are a combination of hardware and software, the software being used to configure the hardware and/or processor.

Hereinafter, an embodiment of the present disclosure will be described in detail with reference to the accompanying drawings.

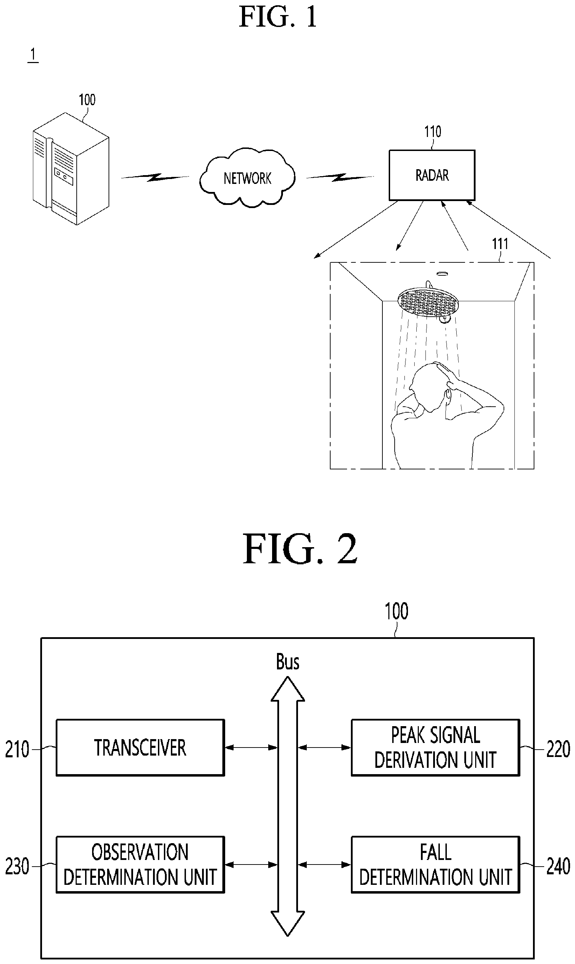

**FIG. 1** is a configuration diagram of a fall detection system according to an embodiment of the present disclosure.

Referring to **FIG. 1,** a fall detection system 1 may include a fall detection device 100 and a radar 110.

The components of the fall detection system 1 illustrated in **FIG. 1** are typically connected to each other via a network. For example, as illustrated in **FIG. 1,** the fall detection device 100 and the radar 110 may be connected simultaneously or sequentially.

The network refers to a connection structure that enables information exchange between nodes such as devices, devices, etc. and includes LAN (Local Area Network), WAN (Wide Area Network), Internet (WWW:

World Wide Web), a wired or wireless data communication network, a telecommunication network, a wired or wireless television network, and the like. Examples of the wireless data communication network may include 3G, 4G, 5G, 3GPP (3rd Generation Partnership Project), LTE (Long Term Evolution), WIMAX (World Interoperability for Microwave Access), Wi-Fi, Bluetooth communication, infrared communication, ultrasonic communication, VLC (Visible Light Communication), LiFi, and the like, but may not be limited thereto.

The fall detection device 100 may analyze a radar signal reflected from a subject 111 by using the radar 110. The fall detection device 100 may detect the presence or absence of a fall of the subject 111 based on a radar signal which changes when the subject 111 falls during indoor activities (e.g., in a shower room, a bathroom, a workroom, and a corridor). For example, the fall detection device 100 may be installed at a ceiling of the shower room so as to be spaced apart from the subject 111 taking a shower, and thus may transmit a radar signal toward the subject 111 and receive a radar signal reflected from the subject 111 through the radar 110.

The fall detection device 100 may determine the presence or absence of the subject 111, the presence or absence of a fall, and the like by using the radar 110.

Therefore, the fall detection device 100 enables a precise determination on the presence or absence of the subject 111 or the presence or absence of a fall even when the subject 111 does not hold or wear a separate device for detecting a fall.

The radar 110 may be attached to or mounted on the fall detection device 100 to detect a fall of the subject 111. Also, at least some components of the fall detection device 100 may be located in a space separate from the radar 110, and may communicate by wire or wirelessly with the radar 110 through the network to detect a fall of the subject 111.

Hereinafter, each component of the fall detection device 100 will be described.

**FIG. 2** is a configuration diagram of a fall detection device according to an embodiment of the present disclosure.

Referring to **FIG. 2,** the fall detection device 100 may include a transceiver 210, a peak signal derivation unit 220, an observation determination unit 230, and a fall determination unit 240. However, these components 210 to 240 are just examples of components that can be controlled by the fall detection device 100.

The transceiver 210 may transmit a radar signal toward a subject and receive a radar signal reflected from the subject.

The peak signal derivation unit 220 may derive a peak signal by filtering the radar signal.

The observation determination unit 230 may determine whether or not to enter an observation state of the subject by analyzing the received radar signal.

The fall determination unit 240 may derive a range value between the transceiver 210 and the subject in the observation state, and determine whether the subject has fallen based on the range value.

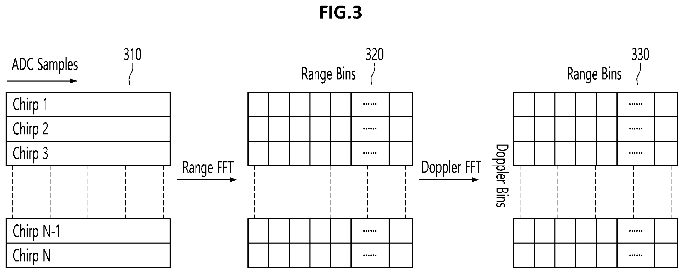

**FIG. 3** is a diagram explaining a procedure of generating range-Doppler map information according to an embodiment of the present disclosure.

**FIG. 3** shows the procedure of generating the range-Doppler map information from the radar signal by the peak signal derivation unit 220 to derive a peak signal based on the radar signal. The peak signal derivation unit 220 may generate the range-Doppler map information through a preprocessing process of processing the radar signal as raw data obtained from the radar.

The peak signal derivation unit 220 may receive the radar signal reflected from the subject and sample each chirp of the received radar signal by an analog-to-digital converter (ADC) to generate a digital signal 310. In this process, the sampled data for each chirp form the structure of a raw radar signal.

Then, the peak signal derivation unit 220 may apply a two-dimensional Fast Fourier Transform (2D-FFT) to the digital signal. Through the 2D-FFT, the digital signal may be transformed from a time domain to a range-Doppler domain. A first Fourier transform may be performed to extract range information of each subject by transforming a time domain signal to a range domain signal 320, and a second Fourier transform may be performed to obtain velocity information 330 of the subject by analyzing a change in Doppler frequency for each range.

In the range-Doppler map, the intensity of a signal depending on the range may be plotted on the horizontal axis, and the Doppler frequency may be plotted on the vertical axis.

The transformed range-Doppler map information shows range and velocity information of the subject from which the radar signal is reflected as two-dimensional images, and it can provide important information for detecting a specific event, such as a fall. Further, the range-Doppler map information is analyzed by a fall detection algorithm and used to determine whether a fall occurs.

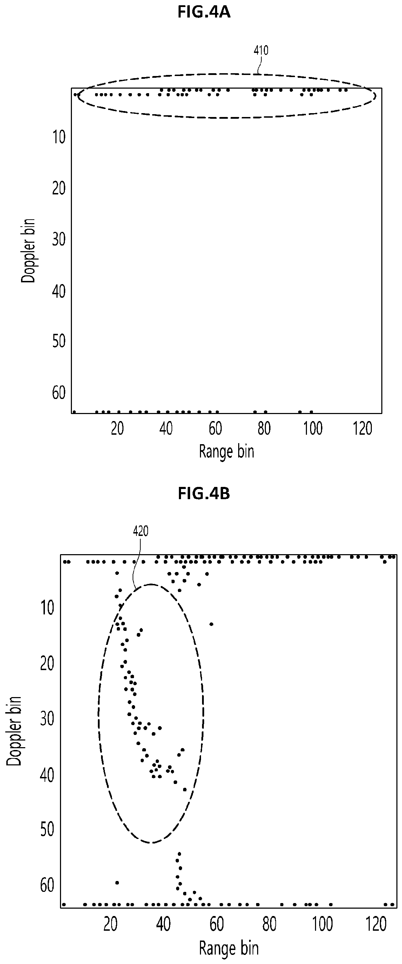

**FIG. 4A** and **FIG. 4B** are diagrams explaining a procedure of deriving a peak signal according to an embodiment of the present disclosure.

The peak signal derivation unit 220 may generate range-Doppler map information based on the radar signal and derive the peak signal by filtering the range-Doppler map information. Herein, the range-Doppler map information may be generated for each frame corresponding to a time point when the transceiver 210 receives the radar signal. That is, the range-Doppler map information may include at least one of a plurality of range-Doppler maps corresponding to respective frames.

The peak signal derivation unit 220 may remove noise from the range-Doppler map and derive a signal with an intensity equal to or higher than a predetermined threshold value as the peak signal.

Herein, the peak signal derivation unit 220 may derive the peak signal by applying a constant false alarm rate (CFAR) detection algorithm. The peak signal derivation unit 220 may analyze the level of ambient background noise around the subject 111 by using the CFAR detection algorithm. Therefore, it is possible to select an ambient region instead of the subject and compute an average signal intensity of the selected regio. Then, the peak signal derivation unit 220 may dynamically determine a threshold value for differentiating a signal of the subject from noise based on the computed average noise level. Herein, the threshold value is adjusted in proportion to the level of ambient background noise, and, thus, signal detection performance can be uniformly maintained in various environments.

The peak signal derivation unit 220 may also apply a threshold value using histogram in addition to the CFAR detection algorithm to derive a peak signal in the range-Doppler map.

The peak signal derived by the peak signal derivation unit 220 may be filtered in terms of power, range, and Doppler to obtain a desired peak signal which can be used as feature information for pattern recognition.

Then, the peak signal derivation unit 220 may scan the entire range-Doppler map and regard a signal, which exceeds a predetermined threshold value, as the peak signal. Through this process, it is possible to derive a peak signal corresponding to location and velocity information of the subject 111.

FIG. 4A illustrates an example of the range-Doppler map information generated when the subject falls, and FIG. 4B illustrates an example of the range-Doppler map when a certain movement (e.g., dribbling of water sprayed from a shower head on the floor of an indoor space) instead of a fall occurs in a space equipped with the fall detection device.

Each of FIG. 4A and FIG. 4B illustrate one frame of the plurality of range-Doppler maps corresponding to respective frames. For example, FIG. 4A may be a range-Doppler map for one of frames corresponding to a time point when the subject falls. Further, FIG. 4B may be a range-Doppler map for one of frames corresponding to a time point when the subject does not fall, but water falls while the subject takes a shower. In FIG. 4A and FIG. 4B, peak signals may be indicated by dots.

It can be seen from FIG. 4A that peak signals 410 are shown in a relatively narrow Doppler bin region and in a wide range bin region when a fall occurs. It can be seen from FIG. 4B that peak signals 420 are shown in a relatively wide Doppler bin region and in a narrow range bin region when other movements than a fall occur.

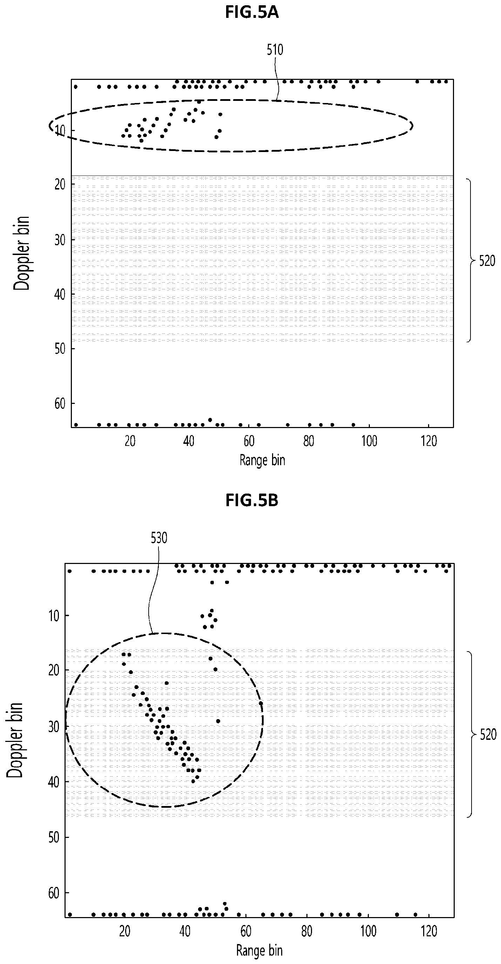

FIG. 5A and FIG. 5B are diagrams explaining a procedure of deriving a movement peak signal according to an embodiment of the present disclosure.

The observation determination unit 230 may derive a movement peak signal relevant to a movement of the subject 111 from the radar signal, analyze a pattern of movement peak signals, and determine whether or not to enter an observation state based on a result of pattern analysis.

Herein, the movement peak signal may be derived by filtering at least a part of the Doppler bin region of the radar signal or based on a distribution of the radar signal.

As described above with reference to FIG. 4A and FIG. 4B, it can be seen that a distribution or pattern of peak signals varies depending on the presence or absence of a fall of the subject 111. FIG. 5A illustrates an example of a distribution of peak signals 510 in the situation shown in FIG. 4A. FIG. 5B illustrates an example of a distribution of peak signals 530 in the situation shown in FIG. 4B. The observation determination unit 230 may derive a movement peak signal with a high relevance to a movement of the subject 111 from the peak signals. To this end, the observation determination unit 230 may derive, as a movement peak signal, a peak signal except peak signals corresponding to a part of a Doppler bin region 520 in a range-Doppler map. That is, the observation determination unit 230 may derive only a peak signal relevant to a movement of the subject 111 from, for example, various movements occurring while the subject 111 takes a shower in shower room. To this end, the observation determination unit 230 may derive, as a movement peak signal of the subject, a peak signal corresponding to the other region except the Doppler bin region 520 relevant to movements of water. The Doppler bin region 520 may be arbitrarily determined based on the intent of the designer or the experimentally derived values.

Meanwhile, the observation determination unit 230 may identify the distribution or pattern of peak signals depending on the situation and filter a peak signal corresponding to noise among the peak signals to derive a movement peak signal.

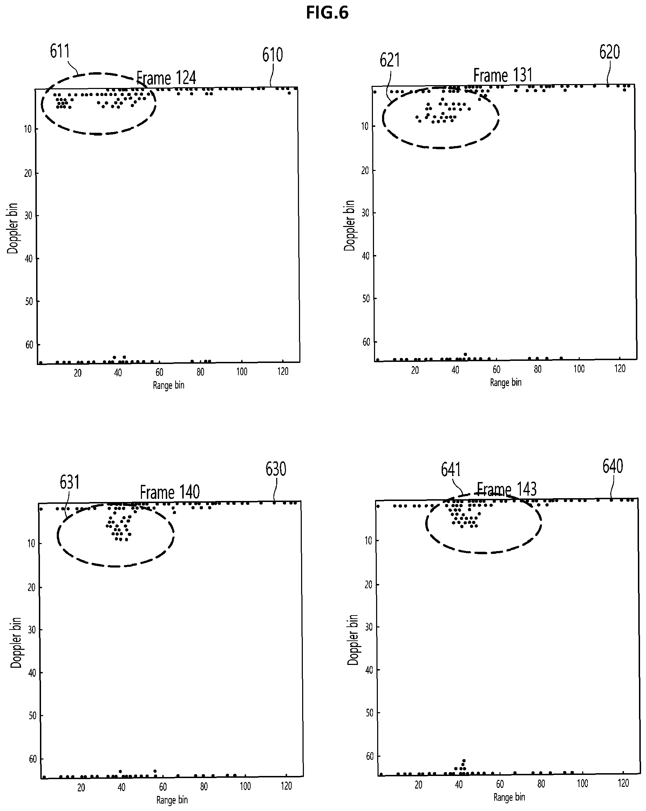

FIG. 6 is a diagram explaining a procedure of analyzing a pattern of movement peak signals according to an embodiment of the present disclosure.

FIG. 6 illustrates examples of range-Doppler maps continuously generated over time. The range-Doppler maps 610, 620, 630 and 640 in FIG. 6 show changes in distribution of movement peak signals 611, 621, 631 and 641 over time when a fall occurs. More specifically, FIG. 6 illustrates an example of the range-Doppler map 610 corresponding to a 124th frame, an example of the range-Doppler map 620 corresponding to a 131st frame, an example of the range-Doppler map 640 corresponding to a 140th frame, and an example of the range-Doppler map 640 corresponding to a 143rd frame.

Referring to each of the range-Doppler maps in FIG. 6, it can be seen that the main distribution of movement peak signals moves from a lower range bin to a higher range bin as time passes. That is, it can be seen that a range bin tends to gradually increase within a certain Doppler bin region as time passes.

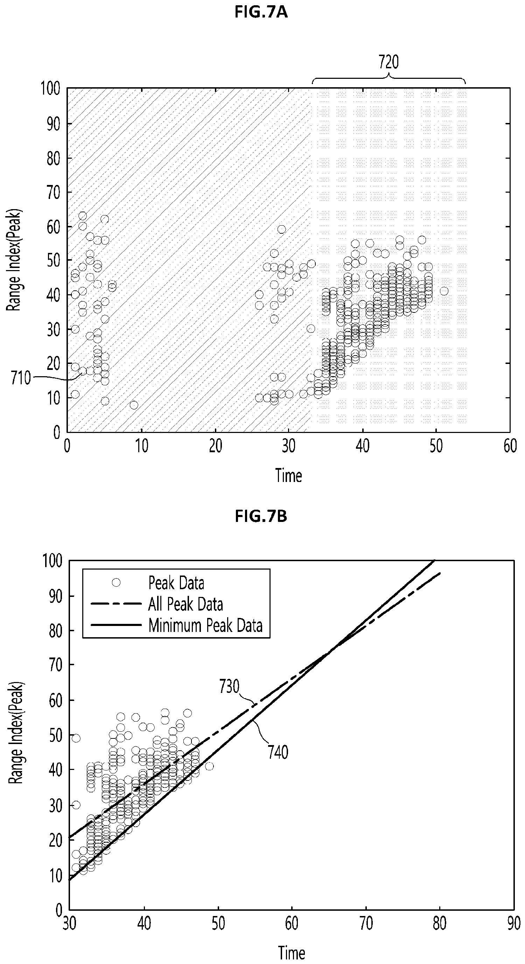

FIG. 7A and FIG. 7B are diagrams explaining a procedure of deriving a trend line according to an embodiment of the present disclosure.

The observation determination unit 230 may derive range indexes of movement peak signals over time and derive a trend line depending on a variance in range index to analyze a pattern of the movement peak signals. Herein, the trend line may be derived from the variance in range index by applying a least square method.

Further, the observation determination unit 230 determines to enter the observation state when a slope of the trend line is equal to or higher than a predetermined value.

**FIG. 7A** and **FIG. 7B** show examples of two-dimensional coordinate systems each having time on the horizontal axis and the range index on the vertical axis, and each dot 710 in the coordinate systems may correspond to a movement peak signal. That is, **FIG. 7A** and **FIG. 7B** show distributions of movement peak signals over time. The dots 710 in **FIG. 7A** correspond to range bins of objects moving in a range-Doppler map over time.

Referring to **FIG. 7A,** it can be seen that the movement peak signals are distributed, increasing from a time point when a time value is greater than about 33. That is, it can be determined that a suspicious fall interval 720 starts from the time point when the time value is greater than 33. In **FIG. 7A** and **FIG. 7B,** the unit of the time axis may be second or frame.

**FIG. 7B** shows a trend line derived based on range indexes of respective movement peak signals in the suspicious fall interval 720. A first trend line 730 is a trend line derived based on all the movement peak signals, and a second trend line 740 is a trend line derived from only movement peak signals with the minimum range indexes among the movement peak signals.

Due to the characteristics of a radar, data received through multiple reflections of electromagnetic waves may include several peak signals even for a single target. For more accurate analysis, the following description is made on the assumption that the second trend line is used.

The observation determination unit 230 may derive a trend line in consideration of a distribution of movement peak signals from the time-range index coordinate system. The trend line may be derived based on the fact that a distance between the radar and the subject increases when the subject falls. The observation determination unit 230 may use these features to suspect that the subject has fallen and determine to enter the observation state to be escribed later.

A slope of the trend line may be derived based on the following equation.

<Equation 1>

$$a = \frac{\sum_i (x_i - \bar{x})(y_i - \bar{y})}{\sum_i (x_i - \bar{x})^2}$$

$$b = \bar{y} - a\bar{x}$$

Herein, a denotes a slope of the trend line, x denotes a time value, and y denotes a range index value.

An increase in slope of the trend line in a positive direction means a more rapid increase in distance between the subject and the radar, which suggests that a fall is likely to occur. Therefore, the observation determination unit 230 may suspect the presence or absence of a fall when the slope of the trend line is higher than a predetermined value, and thus may determine to enter the observation state to be escribed later. Herein, a reference value of the slope of the trend line as a reference for entry into the observation state may be arbitrarily determined based on the intent of the designer or the experimentally derived values.

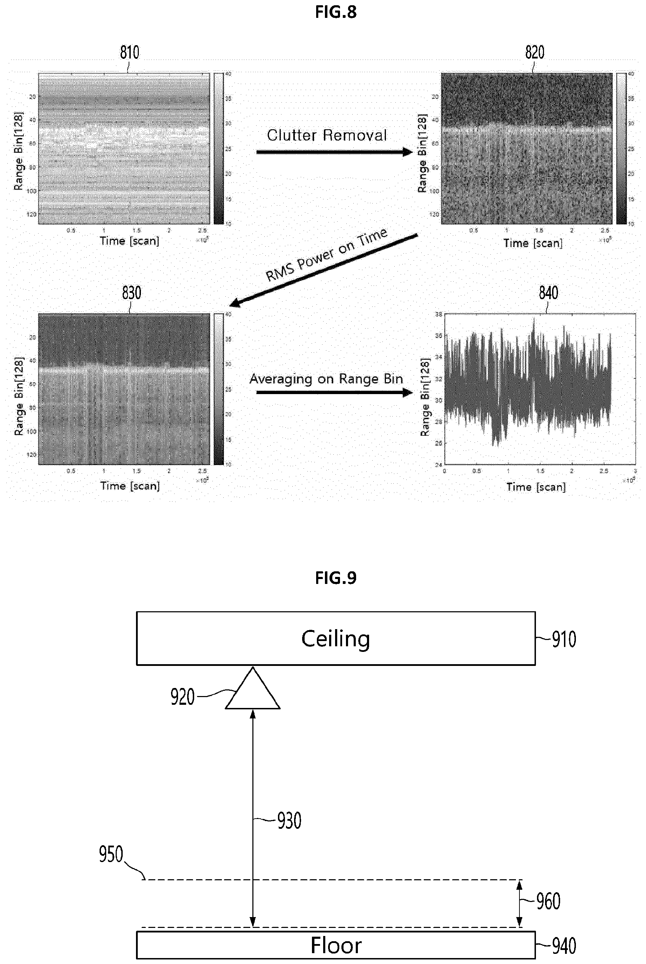

**FIG. 8** is a diagram explaining a procedure of deriving a range value according to an embodiment of the present disclosure.

The fall determination unit 240 may derive a range value between the radar and the subject in the observation state, derive a fall range depending on a height of the space equipped with the radar, and determine whether the subject has fallen based on the range value and the fall range.

Herein, the range value may be obtained by filtering a signal corresponding to a movement of the subject from the radar signal and deriving a root mean square (RMS) power derived from the filtered signal while shifting a window corresponding to a time domain.

The fall range may refer to a range between a far distance threshold point corresponding to a distance from the radar to the floor of the space and a near distance threshold point corresponding to a position spaced apart by a predetermined distance from the far distance threshold point toward the radar.

The fall determination unit 240 may determine the presence or absence of a fall when the range value is lower than a presence threshold point or an average distance is higher than an absence threshold point. The presence threshold point may be derived based on the range value derived when the subject is present in the space, and the absence threshold point may be derived based on the range value derived when the subject is not present in the space.

**FIG. 8** illustrates an example of a procedure of deriving a range value by the fall determination unit 240 in the observation state. The fall determination unit 240 may derive a range bin value 810 derived from the radar signal on a time basis. The range bin value 810 corresponds to a result of time sequentially plotting a signal power on the horizontal axis of a zero Doppler region where immobile objects are present. The immobile objects need to be measured at a predetermined power despite a lapse of time. However, if there is another object showing a slight movement, the zero Doppler region is changed by the movement of the object.

Then, the fall determination unit 240 may derive a result 820 of performing first filtering by extracting only a magnitude of a zero Doppler signal from the derived range bin value 810 and removing a useless component for a virtual detection system, such as an immobile target or clutter in the time domain. Through the clutter removal, changes in signal over time can be detected from the zero Doppler region.

Thereafter, the fall determination unit 240 may output a result 830 of deriving an RMS power on the time domain with respect to the result 820 of performing the first filtering. Further, the fall determination unit 240 may output a result 840 of deriving an average distance, *i.e.*, a range value, based on the range bin with respect to the derived result 830.

Hereinafter, the procedure (810 to 840) of deriving the range value by the fall determination unit 240 will be described in more detail.

The fall determination unit 240 may extract a signal power from a zero Doppler signal, which is a reflect signal when the subject does not move relative to the radar, *i.e.*, at a velocity of 0. Also, the fall determination unit 240 may remove clutter for filtering a component corresponding to a background signal or noise in order to analyze only a changing part of the radar signal. Thus, the fall determination unit 240 may remove the background signal from the derived range bin value 810 except only a vibrating or moving object, such as the body. The process of removing the clutter may be defined by the following equation.

<Equation 2>

$$y[i] = x[i] - x[i-1] + \alpha y[i-1]$$

Herein, y[i] denotes a value obtained by removing clutter at a time point i, x[i] denotes a magnitude of an input signal at the time point i, and $\alpha$ denotes a tuning parameter of the system. More specifically, x[i] denotes an input signal at a current time point and x[i-1] denotes an input signal at a previous time point. An output signal may be obtained by removing clutter according to Equation 2.

Then, the fall determination unit 240 may set a window size corresponding to a certain period in the time domain and compute an RMS power within each window while shifting the set window (windowing). The RMS power is an average power of radar signals, and may reflect changes in signal over time.

The process of obtaining the RMS power may be defined by the following equation.

<Equation 3>

$$y[i] = \sqrt{\frac{1}{N}\sum_{n=0}^{N-1} x[i-n]^2}$$

Herein, y[i] denotes an RMS power output at a time point i and x[i] denotes a value input at the time point i. Herein, x[i] may be a value (y[i] in Equation 2) obtained by removing clutter as derived by Equation 2. Further, N denotes a value corresponding to the window size. With the window size N set for the time domain, it is possible to compute an average of squared input signal values. Then, an RMS power value can be obtained by obtaining a square root of the computed average. That is, the RMS power value indicating an average magnitude of signals can be derived by Equation 3.

Thereafter, the fall determination unit 240 may calculate an average RMS power on the range bin in order to compute an average intensity of signals in each range.

The process of calculating the average RMS power may be defined by the following equation.

<Equation 4>

$$y[i] = C \sum_{n=4}^{128} x[i,n]$$

Herein, y[i] denotes an average RMS power at a time point i and x[i, n] denotes an RMS power corresponding to an nth range bin at the time point i.

Finally, the fall determination unit 240 may compute a range value by applying a Gaussian distribution to the derived average RMS power. Through this process, the fall determination unit 240 may obtain a range value of the subject. Since the range value is derived by using an average value of the Gaussian distribution, an average distance between the subject and the radar can be estimated more accurately.

The process of deriving the range value may be defined by the following equation.

<Equation 5>

$$y[i] = \frac{\sum_{n=4}^{128} x[i,n]^2 \cdot n}{\sum_{n=4}^{128} x[i,n]^2}$$

Herein, y[i] denotes a range value at a time point i and x[i, n] denotes an RMS power corresponding to an nth range bin at the time point i.

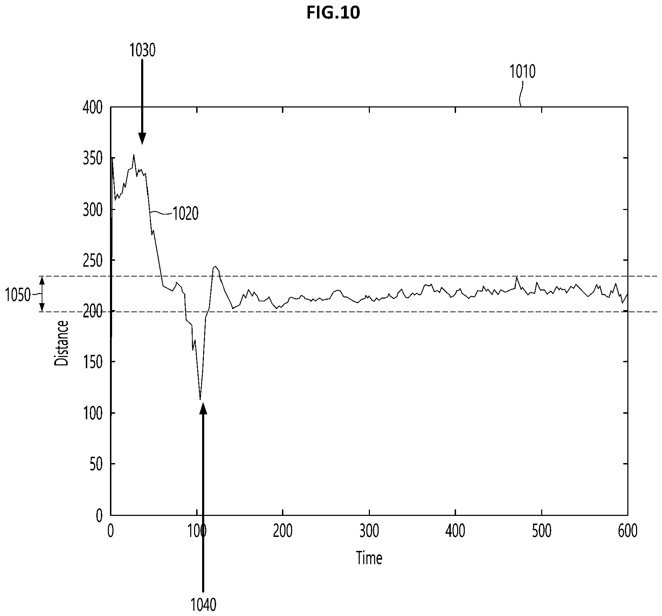

**FIG. 9** and **FIG. 10** are diagrams explaining a procedure of determining the presence or absence of a fall according to an embodiment of the present disclosure.

**FIG. 9** illustrates an example where a radar 920 of the fall detection device 100 is installed at a ceiling 910 of an indoor space. The radar 920 of the fall detection device 100 may transmit a radar signal toward a floor 940 of the indoor space and receive a reflected radar signal. In this case, a distance between the ceiling 910 equipped with the radar 920 and the floor 940 may be defined as a height 930 of the indoor space. A fall range 960 may be defined by a range between a position 950 spaced apart by a predetermined distance from the floor 940 and the floor 940. When a position of the subject derived from the range value of the subject is within the fall range 960 for a predetermined period of time, the fall detection device 100 may determine that the subject has fallen.

In the process of deriving the fall range 960, the floor 940 may be defined as a far distance threshold point, and a height of the position 950 spaced apart by the predetermined distance from the floor 940 may be defined as a near distance threshold point. Herein, the height of the near distance threshold point may be arbitrarily determined based on the intent of the designer or the experimentally derived values.

**FIG. 10** illustrates an example of a graph 1010 derived from a range value 1020 between the radar and the subject, which is derived by the fall determination unit 240, over time. It can be seen from **FIG. 10** that the range value 1020 is from 300 to 350 at a first time point 1030 and then decreases, and is from 100 to 150 at a second time point 1040. Herein, the first time point 1030 refers to a time point when the subject enters the indoor space equipped with the radar, and the second time point 1040 refers to a time point when the subject falls. **FIG. 10** illustrates an example where the range value 1020 of the subject is changed within a certain range after the second time point 1040 when the subject falls. When the range value 1020 of the subject is within a fall range 1050 for a predetermined period of time, the fall determination unit 240 may determine that the subject is in a fallen state.

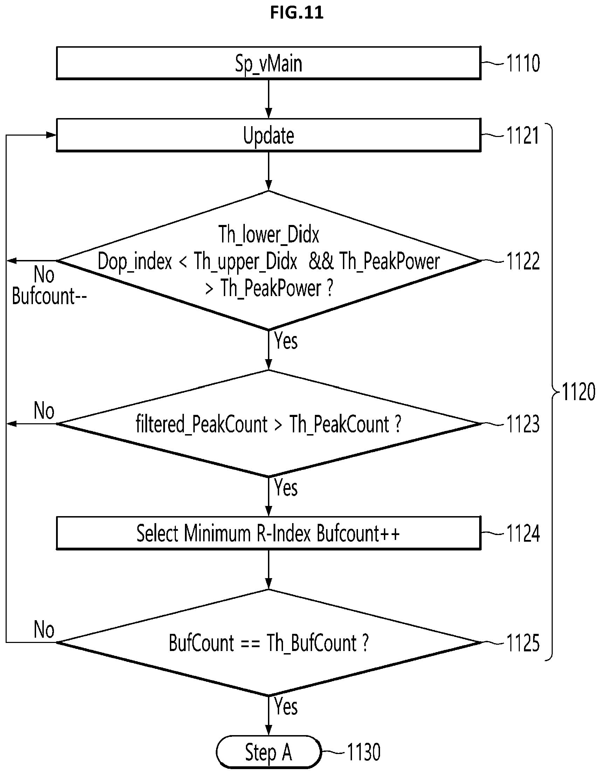

**FIG. 11** to **FIG. 13** are flowcharts showing a fall detection method according to an embodiment of the present disclosure.

**FIG. 11** to **FIG. 13** are flowcharts provided to explain the above-described procedure of detecting a fall of the subject by the fall detection device 100 in more detail with reference to the predefined parameters for the convenience of description.

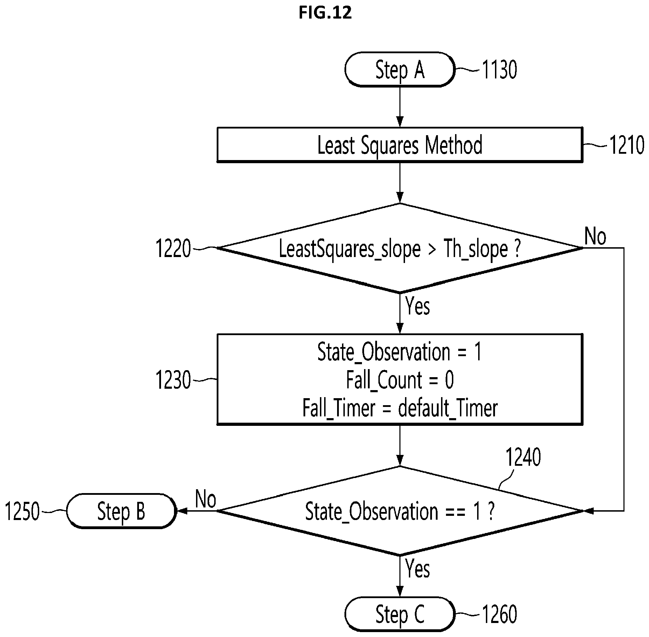

**FIG. 11** and **FIG. 12** illustrate examples of operations performed by the peak signal derivation unit 220 and the observation determination unit 230 based on a radar signal received from the transceiver 210 of the fall detection device 100.

The fall detection device 100 may initialize variables and execute a main function Sp_vMain based on a radar signal received from the transceiver 210 in a process 1110. In this case, the variables may be initialized as follows: State_Observation = 0; BufCount=0; Fall Timer=FALLDET_FALL TIMER; and Fall_Count=0.

Herein, State_Observation denotes a parameter presenting an observation state, and may refer to information on whether or not to enter the observation state with a value of 0 or 1. BufCount denotes a variable used in the fall detection algorithm, and may refer to a value which increases when a movement suspected as a fall occurs on each scan by the radar and decreases in the contrary case. Fall_Timer denotes a parameter for time of observation state, and its value decreases on each scan by the radar in the observation state and if FallCount is not equal to Th_FallCount until the value reaches 0, it may be determined that a fall has not occurred and the observation state may be ended. FallCount may increase or decrease depending on the situation in the observation state.

The fall detection device 100 may generate and update range-Doppler map information from a radar signal acquired from the transceiver 210 at each radar scan and derive a peak signal in a process 1121.

The fall detection device 100 may derive a movement peak signal from the peak signal by filtering the updated range-Doppler map information in a process 1122. Herein, the process of deriving the movement peak signal may be performed when a Doppler index value Dop_Index satisfies a range between Th_lower_Didx and Th_upper_Didx and it is checked whether a peak signal power PeakPower) is higher than Th_PeakPower.

Herein, each of Th_lower_Didx and Th_upper_Didx may be a parameter for removing movements irrelevant to a fall (*e.g.*, movements of water) in an algorithm for detecting a fall, and may be defined by a predetermined Doppler index value.

Th_PeakPower may be a parameter for removing movements of other objects than the subject, and may be defined based on a predetermined decibel (dB).

If the conditions are not satisfied, the process returns back to the initial process 1121, and if the conditions are satisfied, the process proceeds to a process 1123.

In the process 1123, the fall detection device 100 checks whether the number of movement peaks signals filtered_PeakCount is greater than a predetermined threshold number of movement peaks signals Th_PeakCount, and if the conditions are satisfied, the process proceeds to a process 1124, and, if not, Buf_Count is decreased and the process returns back to the initial process 1121.

In the process 1124, the fall detection device 100 may select a method of selecting a minimum value of a plurality of filtered range-index (R-index) data, store the range-index data with the minimum value, and increase a value of BufCount (process 1124).

The fall detection device 100 checks whether the value of BufCount reaches a predetermined value of Th_BufCount in a process 1125, and if it reaches the value of Th_BufCount, the process proceeds to a process 1130, and, if not, BufCount is decreased and the process returns back to the initial process 1121.

The process 1130 and its subsequent processes will be described with reference to **FIG. 12.**

After the process 1130, the fall detection device 100 may derive a trend line by the least squares method based on the range-index data with the minimum value stored in the process 1210.

The fall detection device 100 may compare a slope LeastSquares_slope of the derived trend line with Th_slope in a process 1220. Herein, Th_slope is a parameter relevant to a slope of a trend line derived by the least squares method, and may be used as a threshold point when a trend line with a higher slope than the parameter is derived. As a value of Th_slope increases, the sensitivity for detecting a fall may decrease. If the conditions are satisfied, the fall detection device 100 allows the process to proceed to a process 1230, and, if not, the fall detection device 100 allows the process to proceed to a process 1240.

In the process 1230, the fall detection device 100 may set conditions for entry into the observation state by adjusting a value of State_Observation, and initialize FallCount and Fall_Timer, which are parameters used in the observation state.

In the process 1240, the fall detection device 100 may check the value of State_Observation to determine whether or not to enter the observation state. If the value of State_Observation is a value (*e.g.*, 1) for entry into the observation state, the process proceeds to a process 1260, and, if not, BufCount is decreased and the process proceeds to a process 1250. Herein, the process 1250 may refer to the process 1121 shown in **FIG. 11.**

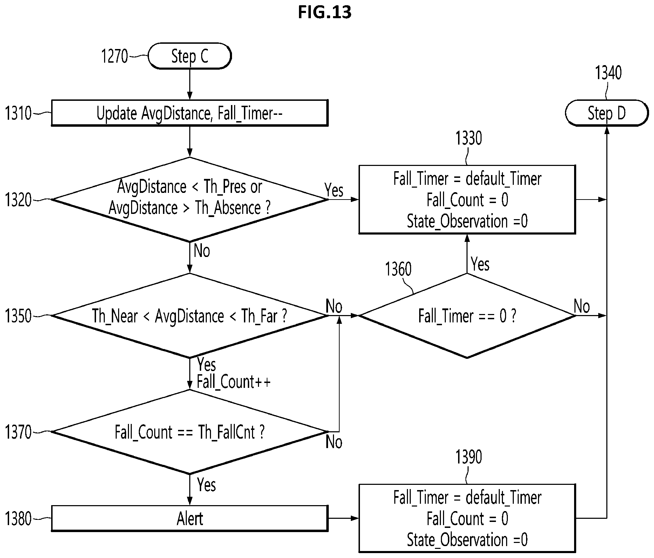

The process 1260 and its subsequent processes will be described with reference to **FIG. 13.**

**FIG. 13** illustrates an example of an operation performed by the fall determination unit 240 of the fall detection device 100 in the observation state.

The fall detection device 100 may update relevant data at each radar scan in a process 1310 subsequent to a process 1270. Herein, the updated data may be a range value AvgDistance of the subject, and whenever the range value AvgDistance is updated, a value of Fall_Timer may be decreased.

The fall detection device 100 may check whether the range value AvgDistance is lower than a presence threshold point Th_Pres or higher than an absence threshold point Th_Absence in a process 1320. Herein, the presence threshold point Th_Pres may refer to a threshold point when a subject is present in an indoor space equipped with a radar and performs other normal activities than a fall. The presence threshold point Th_Pres may be defined based on an average of range values measured when the subject is present in the indoor space. The absence threshold point Th_Absence may refer to a threshold point when nobody is present in the indoor space equipped with the radar. The absence threshold point Th_Absence may be defined based on an average of range values measured when the subject is not present in the indoor space.

If the conditions of the process 1320 are satisfied, the fall detection device 100 allows the process to proceed to a process 1330. The fall detection device 100 initializes FallTimer and FallCount and determines not to enter the observation state in the process 1320, and then performs a process 1340. If not, the fall detection device 100 may perform a process 1350.

Herein, the process 1340 may refer to the process 1121 shown in **FIG. 11.**

In the process 1350, the fall detection device 100 may check whether the range value AvgDistance of the subject is higher than a near distance threshold point Th_Near or lower than a far distance threshold point Th_Far.

The near distance threshold point Th_Near and the far distance threshold point Th_Far will be described in more detail below.

If the conditions of the process 1350 are satisfied, the fall detection device 100 increases FallCount and performs a process 1370. If not, the fall detection device 100 may perform a process 1360.

In the process 1360, the fall detection device 100 checks whether a value of Fall_Time is equal to a predetermined value (*e.g.*, 0). If the conditions of the process 1360 are satisfied, the fall detection device 100 performs the process 1330. If not, the fall detection device 100 may perform the process 1340.

In the process 1370, the fall detection device 100 may check whether FallCount is equal to Th_Fall-Count. Herein, Th_FallCount may be a parameter for finally determining the presence or absence of a fall. When FallCount reaches Th_FallCount, the fall detection device 100 may finally determine that the subject is in a fallen state.

If the conditions of the process 1370 are not satisfied, the fall detection device 100 may perform the process 1360, but if the conditions of the process 1370 are satisfied, the fall detection device 100 may perform a process 1380.

In the process 1380, the fall detection device 100 may determine that the subject is in a fallen state and output relevant information. For example, the fall detection device 100 may transmit a message or signal indicating that the subject is in a fallen state to an external device connected to the fall detection device 100.

Then, the fall detection device 100 initializes Fall_Timer and FallCount and determines not to enter the observation state in a process 1390. Thereafter, the fall detection device 100 may perform the process 1340.

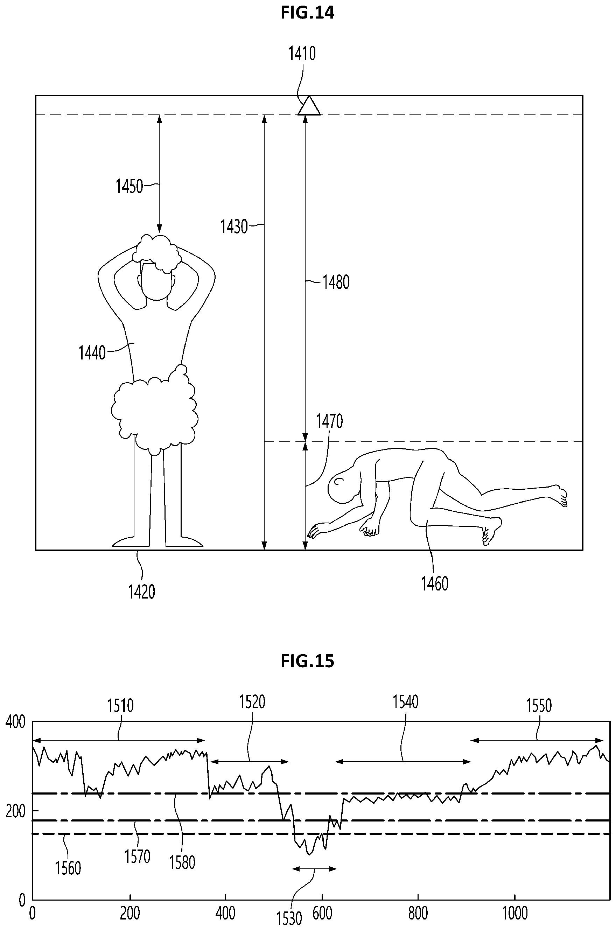

**FIG. 14** and **FIG. 15** are diagrams explaining parameters used in the procedure of determining the presence or absence of a fall according to an embodiment of the present disclosure.

**FIG. 14** is a diagram explaining the near distance threshold point Th_Near, the far distance threshold point Th_Far, and the presence threshold point Th_Pres among the above-described parameters.

**FIG. 14** illustrates that a ceiling of an indoor space is equipped with a radar 1410. In this case, a distance from a height of the radar 1410 to a floor 1420 may be assumed as a height 1430 of the indoor space. If a subject 1440 is present in the indoor space, a distance 1450 between the top of the subject's head and the radar 1410 may be derived as a range value by the fall detection device 100. Herein, the presence threshold point Th_Pres may be derived based on an average of range values 1450 measured when the subject 1440 is present in the indoor and performs other normal activities than a fall.

Further, when a distance from a position of the radar 1410 to the floor 1420 is referred to as the height 1430 of the indoor space, a range value corresponding to the height 1430 of the indoor space may be defined as the far distance threshold point Th_Far. Furthermore, the near distance threshold point Th_Near may be defined based on a distance 1480 from the position of the radar 1410 to a height 1470 of a position spaced apart by a predetermined distance from the floor 1420 in the indoor space. A subject 1460 in a fallen state may be located at a height between the far distance threshold point Th_Far and the near distance threshold point Th_Near.

**FIG. 15** is a graph showing an example of changes in range value depending on the presence or fallen state of the subject. More specifically, **FIG. 15** shows changes in range value during a period 1510 of absence of the subject, a period 1520 of entry of the subject into the indoor space, a period 1530 of presence of the subject, a period 1540 of a fall of the subject 1540, and a period 1550 of absence caused by exit of the subject from the indoor space.

Herein, when a range value lower than a presence threshold point 1560 is derived, the fall detection device 100 may determine that the subject is present in the indoor space. Also, when a range value of the subject is within a range between a near distance threshold point 1570 and a far distance threshold point 1580 for a predetermined period of time, the fall detection device 100 may determine that the subject is in a fallen state.

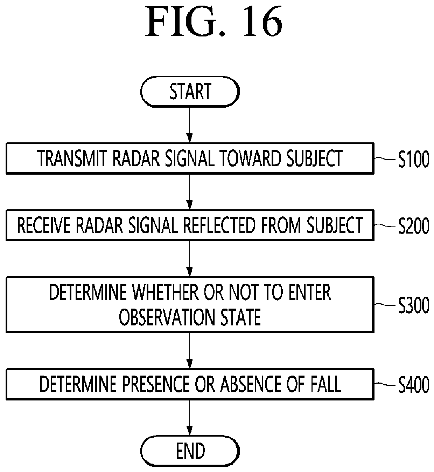

**FIG. 16** is a flowchart showing a fall detection method according to an embodiment of the present disclosure.

The fall detection method using a radar illustrated in **FIG. 16** includes the processes time-sequentially performed according to the embodiment described above with reference to **FIG. 1** to **FIG. 15.** Therefore, the above descriptions of the processes may also be applied to the fall detection method performed by the fall detection device according to the embodiment described above with reference to **FIG. 1** to **FIG. 15** even though they are omitted hereinafter.

Referring to **FIG. 16,** the fall detection method may include a process S100 of transmitting a radar signal toward a subject, a process S200 of receiving a radar signal reflected from the subject, a process S300 of determining whether or not to enter an observation state of the subject by analyzing the received radar signal, and a process S400 of deriving a range value between the transceiver and the subject in the observation state and determining whether the subject has fallen based on the range value.

The fall detection method can be implemented as a computer program stored in a computer-readable storage medium to be executed by a computer or a storage medium including instructions executable by a computer. Also, the fall detection method can be implemented as a computer program stored in a computer-readable storage medium to be executed by a computer.

A computer-readable medium can be any usable medium which can be accessed by the computer and includes all volatile/non-volatile and removable/non-removable media. Further, the computer-readable medium may include all computer storage and communication media. The computer storage medium includes all volatile/non-volatile and removable/non-removable media embodied by a certain method or technology for storing information such as computer-readable instruction code, a data structure, a program module or other data. The communication medium typically includes the computer-readable instruction code, the data structure, the program module, or other data of a modulated data signal such as a carrier wave, or other transmission mechanism, and includes a certain information transmission medium.

The fall detection method may be divided into additional processes or combined into fewer processes in the embodiment described above with reference to **FIG. 1** to **FIG. 15.** In addition, some of the processes may be omitted and the sequence of the processes may be changed if necessary.

The above description of the present disclosure is provided for the purpose of illustration, and it would be understood by those skilled in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

**Claims**

1. A fall detection device of detecting a fall using a radar signal, comprising:

   a transceiver configured to transmit a radar signal toward a subject and receive a radar signal reflected from the subject;
   an observation determination unit configured to determine whether or not to enter an observation state of the subject by analyzing the received radar signal; and
   a fall determination unit configured to derive a range value between the transceiver and the subject in the observation state and determine whether the subject has fallen based on the range value.

2. The fall detection device of Claim 1, further comprising:
   a peak signal derivation unit configured to derive a peak signal by filtering the radar signal.

3. The fall detection device of Claim 2, wherein the peak signal derivation unit generates range-Doppler map information based on the radar signal and derives the peak signal by filtering the range-Doppler map information.

4. The fall detection device of Claim 3, wherein the range-Doppler map information is generated for each frame corresponding to a time point when the radar signal is received.

5. The fall detection device of Claim 3 or 4, wherein the peak signal derivation unit removes noise from the range-Doppler map and derives a signal with an intensity equal to or higher than a predetermined threshold value as the peak signal.

6. The fall detection device of any one of Claims 1 to 5, wherein the observation determination unit derives a movement peak signal relevant to a movement of the subject from the radar signal, analyzes a pattern of movement peak signals, and determines whether or not to enter the observation state based on a result of pattern analysis.

7. The fall detection device of Claim 6, wherein the movement peak signal is derived by filtering at least a part of a Doppler bin region of the radar signal or based on a distribution of the radar signal.

8. The fall detection device of Claim 6 or 7, wherein the observation determination unit derives range indexes of movement peak signals over time and derives a trend line depending on a variance in range index to analyze a pattern of the movement peak signals.

9. The fall detection device of Claim 8, wherein the trend line is derived from the variance in range index by applying a least square method.

10. The fall detection device of Claim 8 or 9, wherein the observation determination unit determines to enter the observation state when a slope of the trend line is equal to or higher than a predetermined value.

11. The fall detection device of any one of Claims 1 to 10, wherein the fall determination unit derives a range value between the transceiver and the subject in the observation state, derives a fall range depending on a height of a space equipped with the transceiver, and determines whether the subject has fallen based on the range value and the fall range.

12. The fall detection device of Claim 11, wherein the range value is obtained by filtering a signal corresponding to a movement of the subject from the radar signal and deriving a root mean square (RMS) power derived from the filtered signal while shifting a window corresponding to a time domain.

13. The fall detection device of Claim 11 or 12, wherein the fall range refers to a range between a far distance threshold point corresponding to a distance from the radar to the floor of the space and a near distance threshold point corresponding to a position spaced apart by a predetermined distance from the far distance threshold point toward the radar.

14. The fall detection device of any one of Claims 11 to 13,

   wherein the fall determination unit determines the presence or absence of a fall when the range value is lower than a presence threshold point or higher than an absence threshold point, and
   the presence threshold point is derived based on the range value derived when the subject is present in the space, and
   the absence threshold point is derived based on the range value derived when the subject is not present in the space.

15. A fall detection method of detecting a fall using a radar signal, comprising:

   a process of transmitting a radar signal toward a subject and receiving a radar signal reflected from the subject by a transceiver;
   a process of determining whether or not to enter an observation state of the subject by analyzing the received radar signal; and

a process of deriving a range value between the transceiver and the subject in the observation state and determining whether the subject has fallen based on the range value.

# FIG. 1

# FIG. 2

**FIG.3**

## FIG.4A

## FIG.4B

**FIG.5A**

**FIG.5B**

**FIG.6**

**FIG.7A**

**FIG.7B**

## FIG.8

## FIG.9

**FIG.10**

**FIG.11**

```
┌─────────────────────────────────────────────────────┐
│                      Sp_vMain                         │──1110
└─────────────────────────────────────────────────────┘
                          │
                          ▼
┌─────────────────────────────────────────────────────┐
│                      Update                           │──1121
└─────────────────────────────────────────────────────┘
                          │
                          ▼
```

Th_lower_Didx  
Dop_index < Th_upper_Didx  && Th_PeakPower  
> Th_PeakPower ?  ──1122

No  
Bufcount--

Yes

filtered_PeakCount > Th_PeakCount ?  ──1123

No

Yes

Select Minimum R-Index Bufcount++  ──1124

BufCount == Th_BufCount ?  ──1125

No

Yes

Step A  ──1130

1120

**FIG.12**

Step A ~1130

Least Squares Method ~1210

1220~ LeastSquares_slope > Th_slope ? — No

Yes

1230~ State_Observation = 1
Fall_Count = 0
Fall_Timer = default_Timer

1240

1250~ Step B — No — State_Observation == 1 ?

Yes

Step C ~1260

**FIG.13**

1270 — ( Step C )

1340

1310 — | Update AvgDistance, Fall_Timer-- |

( Step D )

1320 — ⟨ AvgDistance < Th_Pres or AvgDistance > Th_Absence ? ⟩ — Yes →

1330

| Fall_Timer = default_Timer<br>Fall_Count = 0<br>State_Observation =0 |

No

1350 — ⟨ Th_Near < AvgDistance < Th_Far ? ⟩ — No →

1360

Yes

⟨ Fall_Timer == 0 ? ⟩ — No →

Yes
Fall_Count++

1370 — ⟨ Fall_Count == Th_FallCnt ? ⟩ — No →

Yes

1390

1380 — | Alert | →

| Fall_Timer = default_Timer<br>Fall_Count = 0<br>State_Observation =0 |

**FIG.14**

**FIG.15**

# FIG. 16

```
          ┌──────────┐
          │  START   │
          └──────────┘
                │
                ▼
┌──────────────────────────────────────┐
│  TRANSMIT RADAR SIGNAL TOWARD SUBJECT │─── S100
└──────────────────────────────────────┘
                │
                ▼
┌──────────────────────────────────────┐
│ RECEIVE RADAR SIGNAL REFLECTED FROM   │─── S200
│              SUBJECT                  │
└──────────────────────────────────────┘
                │
                ▼
┌──────────────────────────────────────┐
│    DETERMINE WHETHER OR NOT TO ENTER  │─── S300
│         OBSERVATION STATE             │
└──────────────────────────────────────┘
                │
                ▼
┌──────────────────────────────────────┐
│  DETERMINE PRESENCE OR ABSENCE OF FALL│─── S400
└──────────────────────────────────────┘
                │
                ▼
          ┌──────────┐
          │   END    │
          └──────────┘
```

Europäisches
Patentamt

European
Patent Office

Office européen
des brevets

# EUROPEAN SEARCH REPORT

**Application Number**

EP 24 19 3031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 7 916 066 B1 (OSTERWEIL JOSEF [US]) 29 March 2011 (2011-03-29) | 1,2,6-15 | INV. G01S13/58 |
| Y | * abstract * * column 3, line 14 - column 19, line 40 * ----- | 3-5 | G01S13/88 G08B21/04 |
| Y | ZHANG XUSHENG ET AL: "Waffle", PROCEEDINGS OF THE ACM ON INTERACTIVE, MOBILE, WEARABLE AND UBIQUITOUS TECHNOLOGIES, ACMPUB27, NEW YORK, NY, USA, vol. 7, no. 4, 12 January 2024 (2024-01-12), pages 1-29, XP059190139, DOI: 10.1145/3631458 | 3-5 | |
| A | * abstract * * page 201:2 - page 201:26 * ----- | 1,2,6-15 | |

**TECHNICAL FIELDS SEARCHED      (IPC)**

G01S
G08B

The present search report has been drawn up for all claims

| Place of search | Date of completion of the search | Examiner |
|---|---|---|
| Munich | 17 January 2025 | van Norel, Jan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 19 3031

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2025

| Patent document cited in search report | Publication date | Patent family member(s) | Publication date |
|---|---|---|---|
| US 7916066 B1 | 29-03-2011 | US 7916066 B1 | 29-03-2011 |
| | | US 8068051 B1 | 29-11-2011 |

EPO FORM P0459

For more details about this annex : see Official Journal of the European Patent Office, No. 12/82